# EUROPEAN PATENT SPECIFICATION

(11) **EP 2 554 190 B1**
(45) Date of publication and mention of the grant of the patent: **24.02.2016**
(21) Application number: 11765517.5
(22) Date of filing: 28.03.2011
(51) Int. Cl.: A61M 1/02

(54) **SYSTEM AND METHOD FOR PRIMING LEUKOCYTE REMOVER**
SYSTEM UND VERFAHREN ZUR ENTLÜFTUNG EINES LEUKOZYTENENTFERNUNGSMITTELS
SYSTÈME ET MÉTHODE DE MISE EN CONDITION D'UN SYSTÈME D'ÉLIMINATION DE LEUCOCYTE

(30) Priority: 31.03.2010 JP 2010083452
(43) Date of publication of application: 06.02.2013
(73) Proprietor: Asahi Kasei Medical Co., Ltd., Tokyo 101-8101 (JP)
(72) Inventor: ITO Kozue, Tokyo 101-8101 (JP); SUKEGAWA Takeshi, Tokyo 101-8101 (JP)
(74) Representative: von Kreisler Selting Werner - Partnerschaft von Patentanwälten und Rechtsanwälten mbB
(86) International application number: PCT/JP2011/057677
(87) International publication number: WO 2011/125617

(56) References cited:
- EP-A2- 0 987 034
- WO-A1-02/051520
- CA-A1- 2 183 112
- JP-A- 2000 083 649
- JP-A- 2009 022 762
- JP-A- 2009 050 712
- US-A- 4 925 572
- US-A- 5 690 815

## Description

### Technical Field

The present invention relates to a method for priming a leukocyte remover in which a filter in a substantially dry state is housed.

### Background Art

For the purpose of treating autoimmune diseases such as rheumatism and ulcerative colitis, leukapheresis for selectively removing leukocytes is developed. In the leukapheresis, peripheral blood is collected from a patient using a blood purifier including an extracorporeal circulation circuit configured by a blood collecting circuit, an injection circuit for an anticoagulant agent, a leukocyte remover, a chamber, a blood returning circuit, and the like and a tubing pump, etc., leukocytes in the peripheral blood are selectively removed while the blood is extracorporeally circulated, and the remaining blood components are returned to the patient.

For the purpose of filling a physiological solution to the extracorporeal circulation circuit and removing the air in the extracorporeal circulation circuit, priming is performed before the leukapheresis is started. The structure of the leukocyte remover is most complicated in the extracorporeal circulation circuit. The air tends to remain in the leukocyte remover. A leukocyte remover for extracorporeal circulation in the past is sterilized with filled liquid in a wet state taking into account operability during use and stability of a leukocyte removing material during sterilization (Patent Literature 1). Since liquid is filled on the inside of the leukocyte remover for extracorporeal circulation, an air deflation failure does not occur in priming.

### Citation List

### Patent Literature

Patent Literature 1: Japanese Patent No. 4261623

CA 2,183,112 discloses method and systems for leukocyte depletion using gravity priming with biological fluid.

WO 02/051520 discloses a biological fluid filter capable of filtering a wide variety of biological fluids without being limited by the CWST of the media.

US 4,925,572 relates to devices for the depletion of leukocytes in blood products, preferably comprising an upstream porous element and a downstream element including means for removal of leukocytes by both adsorption and filtration.

US 5,690,815 discloses an automated system for processing biological fluid.

EP 0 987 034 relates fo an apparatus and methods for isolating and recovering cells without deteriorating the quality and characteristics of the cells.

### Summary of Invention

### Technical Problem

In the extracorporeal circulation circuit for performing the leukapheresis, since liquid is filled on the inside of the leukocyte remover, a blood collecting side circuit and a blood returning side circuit are connected to the leukocyte remover immediately before use. However, when efficiency of treatment is taken into account, the extracorporeal circulation circuit is desirably manufactured as an integral extracorporeal circulation circuit to which the blood collecting circuit, the injection circuit for an anticoagulant agent, the leukocyte remover, the chamber, the blood returning circuit, and the like are connected. In order to manufacture the extracorporeal circulation circuit as the integral extracorporeal circulation circuit, the leukocyte remover has to be a dry type. However, a method and a system for priming the leukocyte remover of the dry type are unknown.

It is an object to be solved by the present invention to provide a method and a system for priming a leukocyte remover that can remove, in priming treatment in the filter in a substantially dry state, the air remaining in a container, in which the filter is housed, and prevent deterioration in filter performance.

### Solution to Problem

As a result of earnestly examining the problem in order to solve the problem, the inventors have found that it is possible to prime a leukocyte remover without leaving an amount of the air problematic in treatment if the leukocyte remover is erected, a physiological solution is led into the leukocyte remover from the lower side in the gravity direction without pulsation, and average liquid surface rising speed of the physiological solution is set to be equal to or higher than water absorption speed of a filter. As a result, the inventors have completed the present invention.
(1) A priming method for a leukocyte remover according to the present invention is a method of priming a leukocyte remover in which a filter in a substantially dry state is housed in a container having a first port and a second port for liquid provided near a peripheral edge thereof and a space in the container is partitioned into a first port side space and a second port side space by the filter, the priming method comprising:
   arranging the leukocyte remover such that the second port is located in a lowest position of the leukocyte remover and the first port is located in a highest position of the leukocyte remover by placing the leukocyte remover substantially vertically but whereby the leukocyte remover can be inclined by up to 10 degrees from the vertical direction;
   leading in a physiological solution from the second port; and
   setting the average liquid surface rising speed of the physiological solution in the second port side space to be equal to or higher than the water absorption speed of the filter.

Preferred embodiments of the invention are apparent from the dependent claims

### Advantageous Effects of Invention

According to the present invention, in priming treatment in a filter in a substantially dry state, it is possible to remove the air remaining in a container in which the filter is housed and prevent deterioration in filter performance.

### Brief Description of Drawings

[Figure 1] Figure 1 is a front view of a flat leukocyte remover according to an embodiment of the present invention.
[Figure 2] Figure 2 is a front view of a cylindrical leukocyte remover according to the embodiment of the present invention.
[Figure 3] Figure 3 is a sectional view taken along line III-III in Figure 1.
[Figure 4] Figure 4 is a sectional view taken along line IV-IV in Figure 2.
[Figure 5] Figure 5 is modifications of the flat leukocyte remover according to the embodiment of the present invention, wherein (a) is a front view according to a first modification, (b) is a front view according to a second modification, and (c) is a front view according to a third modification.
[Figure 6] Figure 6 is modifications of the cylindrical leukocyte remover according to the embodiment of the present invention and is a sectional view taken along line VI-VI in Figure 2, wherein (a) is a sectional view according to a first modification, (b) is a sectional view according to a second modification, (c) is a sectional view according to a third modification, and (d) is a sectional view according to a fourth modification.
[Figure 7] Figure 7 is a schematic diagram showing a priming system for a leukocyte remover according to the embodiment of the present invention.

### Description of Embodiments

A preferred embodiment of the present invention is explained in detail below with reference to the drawings. The embodiment explained below does not limit the present invention.

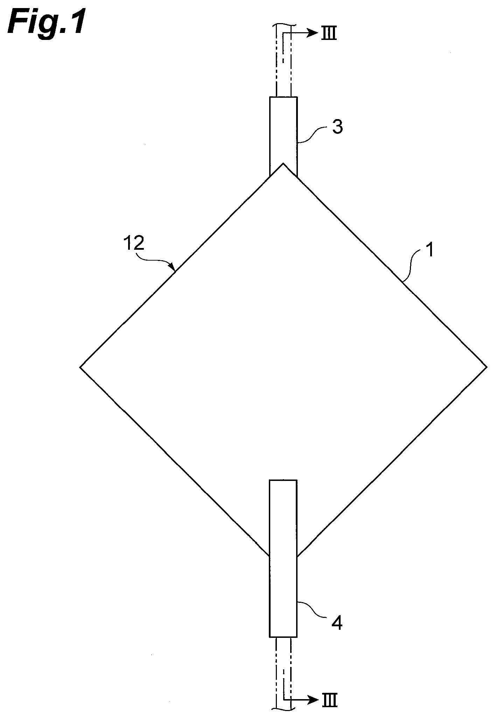

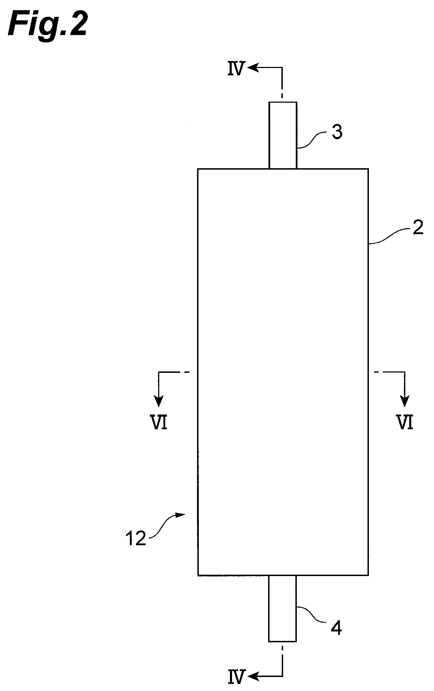

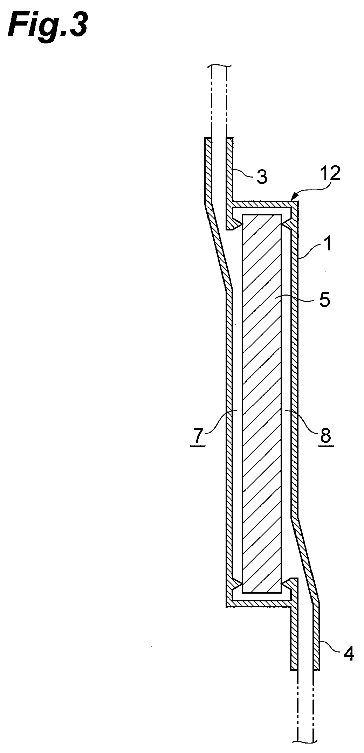

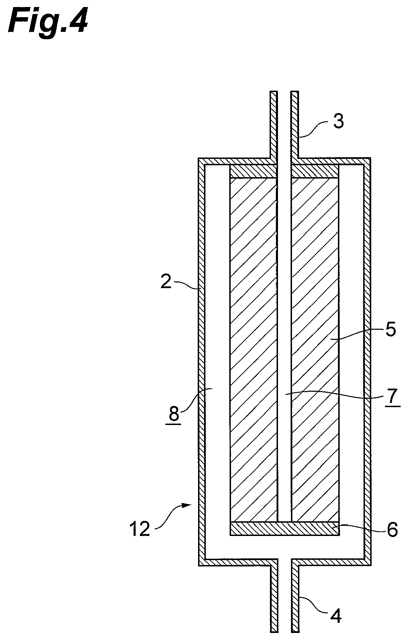

Figures 1 and 2 are schematic front views of a flat leukocyte remover and a cylindrical leukocyte remover according to an embodiment of the present invention. Figures 3 and 4 are sectional views of the flat leukocyte remover and the cylindrical leukocyte remover according to this embodiment. As shown in Figures 3 and 4, a leukocyte remover 12 is a so-called dry type leukocyte remover in which liquid is not filled. A filter 5 in a substantially dry state is housed on the inside of the leukocyte remover 12. The filter 5 in the dry state means a filter not immersed in filled liquid. The filter 5 is housed in a flat container 1 or a cylindrical container 2 including a first port 3 and a second port 4. A space in the flat container 1 or the cylindrical container 2 is partitioned into a first port side space 7 and a second port side space 8 by the filter 5.

The filter 5 is not specifically limited as long as the filter 5 is a component including pores that can filter blood. The filter 5 includes a component having any component form. However, specifically, a fibrous medium such as nonwoven fabric or woven fabric, a porous film, or a porous medium having three-dimensional mesh continuous holes is particularly desirable. A component is not specifically limited as long as the component less easily damages blood corpuscles. Various components can be used. Examples of the components include an organic polymeric material and an inorganic polymeric material. Among the components, the organic polymeric material is a desirable component because the organic polymeric material is excellent in workability of cutting and the like. Examples of the organic polymeric material include polyester, polyolefin, polyacrylonitrile, polyamide, polystyrene, polymethyl methacrylate, polyvinyl fluoride, polyurethane, polyvinylalcohol, polyvinyl acetal, polysulphone, polyvinylidene fluoride, polytrifluorochlorovinyl, vinylidene fluoride-tetrafluoroethylene copolymer, polyether sulphone, polyacrylate, butadiene-acrylonitrile copolymer, polyether-polyamide block copolymer, ethylene-vinylalcohol copolymer, cellulose, and cellulose acetate. The filter according to the present invention is not limited to the examples. The filter is desirably polyester or polyolefin and particularly desirably polyester.

The first port 3 and the second port 4 of the flat container 1 or the cylindrical container 2 can be both an outlet and an inlet for liquid. For example, during priming, the second port 4 can be used as an inlet for a physiological solution and the first port 3 can be used as an outlet for the physiological solution. During blood treatment, the second port 4 can be used as a blood inlet and the first port 3 can be used as a blood outlet or the first port 3 can be used as the blood inlet and the second port 4 can be used as the blood outlet. The first port 3 communicates with the first port side space 7 and the second port 4 communicates with the second port side space 8. The flat container 1 is a flat-shape container having small thickness and a wide surface. The flat container 1 may be either flexible or nonflexible. The cylindrical container 2 is a columnar or prism container. The cylindrical container 2 may be either flexible or nonflexible.

The flat container 1 according to this embodiment has a square flat shape. The first port 3 and the second port 4 for liquid are provided near the peripheral edge of the container. In particular, the first port 3 and the second port 4 according to this embodiment are respectively provided in opposed corner portions of the flat container 1.

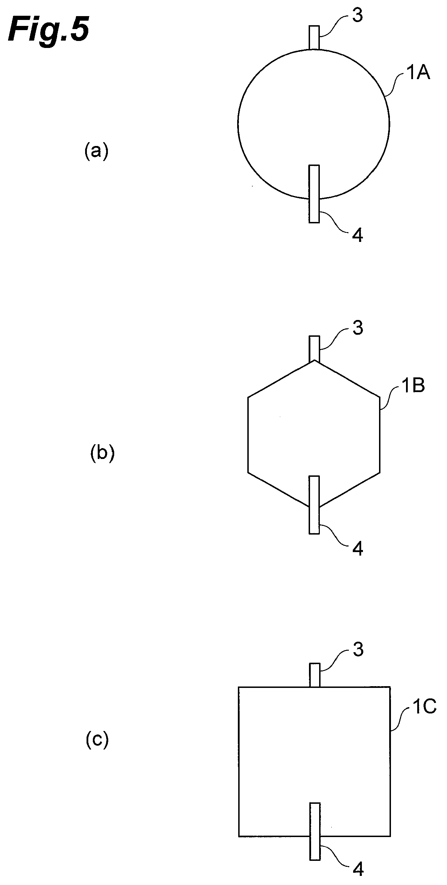

The flat container 1 may have any shape as long as the shape is a flat shape. For example, as a first modification, the flat container 1 can also be a flat container 1A circular in front view (see Figure 5(a)). In the flat container 1A, the first port 3 and the second port 4 can be provided in symmetrical positions of the peripheral edge with reference to the center of the flat container 1A. As a second modification, the flat container 1 can also be a flat container 1B hexagonal in front view (see Figure 5(b)). In the flat container 1B, the first port 3 and the second port 4 can be provided in symmetrical positions at peripheral edge corners with reference to the center of the flat container 1B. As a third modification, the flat container 1 can also be a flat container 1C square in front view (see Figure 5(c)). In the flat container 1C, the first port 3 and the second port 4 can be respectively provided near the centers of a pair of sides opposed to each other across a sheet-like filter among the four sides that form the peripheral edge.

The cylindrical container 2 according to this embodiment is a circular cylindrical container. The first port 3 and the second port 4 for liquid are provided in a surface portion of the container. In particular, the first port 3 and the second port 4 according to this embodiment are respectively provided in the centers of opposed surface portions of the cylindrical container 2.

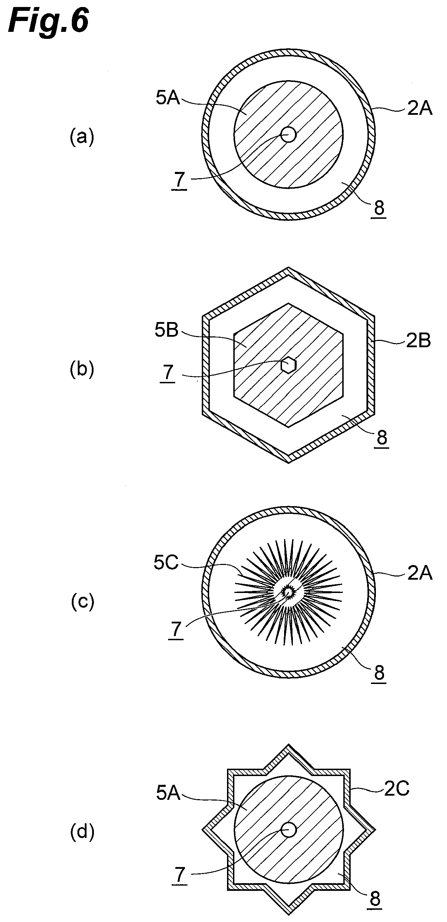

The cylindrical container 2 may have any shape as long as the shape is a cylindrical shape. For example, as a first modification, the cylindrical container 2 can also be a cylindrical container 2A circular in sectional view (see Figures 6(a) and (c)). As a second modification, the cylindrical container 2 can also be a hexagonal shape 2B in sectional view (see Figure 6(b)). As a third modification, the cylindrical container 2 can also be a star-polygonal shape 2C in sectional view (see

Figure 6(d)).

The shape of a cylindrical filter in a substantially dry state housed in the cylindrical container 2 may be any shape as long as the shape is a cylindrical shape. For example, as a first modification, the cylindrical filter can be a filter 5A circular in sectional view (see Figures 6(a) and (d)). As a second modification, the cylindrical filter can also be a filter 5B hexagonal in sectional view (see Figure 6(b)). As a third modification, the cylindrical filter can also be a filter 5C pleats-like in sectional view (see Figure 6(c)).

The shape of the cylindrical filter may be a shape different from the shape of the cylindrical container 2. For example, the pleats-like filter may be housed in the circular cylindrical container (see Figure 6(c)). The cylindrical filter may be housed in the star-polygonal cylindrical container (see Figure 6(d)).

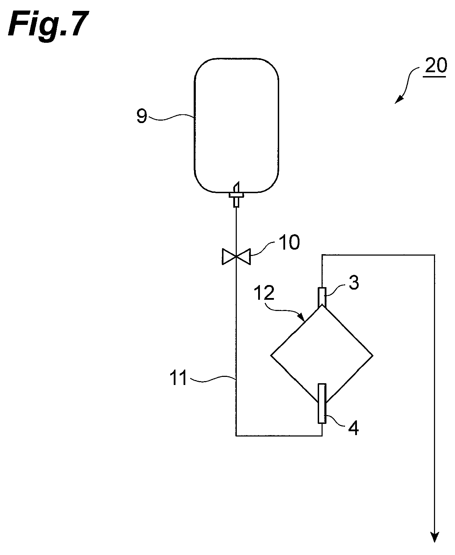

As shown in Figure 7, a priming system 20 includes the leukocyte remover 12, a storing section 9 for a physiological solution arranged in a position higher than the leukocyte remover 12, and a circuit 11 that connects the storing section 9 and the leukocyte remover 12 and transfers the physiological solution. The circuit 11 includes opening and closing means 10 for opening and closing a channel.

The physiological solution according to this embodiment refers to a solution substantially isotonic with body fluid and a solution prepared such that cells and tissues can maintain a physiological state. The physiological solution can be administered to a living body and does not substantially adversely affect the living body. Examples of the physiological solution include physiological salt solutions such as physiological saline, Ringer's solution, lactic acid Ringer's solution, acetic acid Ringer's solution, and tyrode liquid, sugar containing liquid such as glucose, and physiological salt solutions including an anticoagulant agent such as citrate, heparin, or mesylate nafamostat. Among these solutions, the physiological saline and the physiological saline including the anticoagulant agent are desirably used.

The storing section 9 for the physiological solution may be any storing section as long as the storing section can aseptically store the physiological solution. However, a flexible bag such as a transfusion bag is desirable from the viewpoint of handleability.

The circuit 11 includes the opening and closing means 10 for closing and opening a tube or a channel that connects the storing section 9 and the second port 4 of the leukocyte remover 12. The opening and closing means 10 only has to be opening and closing means that can close and open the channel in the circuit 11. A clamp or a roller clamp that can adjust a channel diameter can be used. Besides the opening and closing means 10, a drip chamber or a heating bag may be provided in the circuit 11.

Critical wet surface tension (CWST) of the filter 5 of the leukocyte remover 12 has to be equal to or larger than the surface tension of the physiological solution. The surface tension of the physiological solution is measured using a so-called Wilhelmy method for preparing a platinum plate, the surface of which is suffciently red-heated with an alcohol lamp or the like and cleaned, immersing the platinum plate in the physiological solution, and measuring resistance in drawing out the platinum plate. As a surface tensiometer, for example, FACE SURFACE TENTIOMETER CBVP-A3 manufactured by Kyowa Interface Science Co., Ltd. can be used.

When the surface tension of the physiological solution is measured by this method, for example, the surface tension of physiological saline or physiological saline including an anticoagulant agent such as Citra ph, ACD-A, or Futhan is 72 dyn/cm. Therefore, if priming is performed using the physiological saline or the physiological saline including the anticoagulant agent, the CWST of the filter material has to be equal to or higher than 72 dyn/cm. The CWST of the filter material is desirably equal to or higher than 72 dyn/cm and equal to or lower than 115 dyn/cm and more desirably equal to or higher than 72 dyn/cm and equal to or lower than 100 dyn/cm.

The CWST is a physical property related to a surface characteristic of the filter material and used in specifying a wetting characteristic of the filter material. Specifically, the CWST is a surface characteristic value for specifying whether wetting in the filter material occurs when liquid is brought into contact with the surface of the filter material and pressure is slightly applied to the liquid. In a filter material having CWST larger than the surface tension of certain liquid, wetting by the liquid occurs.

The CWST according to this embodiment refers to a value calculated by a method explained below. Water solutions having different concentrations of sodium hydroxide, calcium chloride, sodium nitrate, acetic acid, and ethanol are prepared such that surface tension changes by 2 to 4 dyn/cm in each of the water solutions. The surface tensions (dyn/cm) of the water solutions are 94 to 115 for the sodium hydroxide water solution, 90 to 94 for the calcium chloride water solution, 75 to 87 for the sodium nitrate water solution, 72.4 for pure water, 38 to 69 for the acetic acid water solution, and 22 to 35 for the ethanol water solution ("Chemical Manual Basic Edition II", second revised edition, edited by The Chemical Society of Japan, Maruzen, 164 (1975)). A water solution having surface tension of 74 to 100 dyn/cm may be prepared from sodium hydroxide. Ten drips each of the water solutions having the difference in surface tensions of 2 to 4 dyn/cm obtained in this way are placed on the filter material in order from the water solution having the lowest surface tension and left untouched for ten minutes. After being left untouched for ten minutes, when nine drips or more of the ten drips are absorbed by the filter material, this is defined as a wet state. When the absorbed drips are less than nine drips among the ten drips, this is defined as a non-wet state. When the liquids are sequentially measured on the filter material from the liquid having the smallest surface tension in this way, the wet state and the non-wet state appear. At this point, an average of values of the surface tensions of the liquids in which the wet state is observed and values of the surface tensions of the liquids in which the non-wet state is observed is defined as a CWST value of the filter material. For example, when a filter material is wet by liquid having surface tension of 64 dyn/cm and not-wet by liquid having surface tension of 66 dyn/cm, a CWST value of the filter material is 65 dyn/cm.

The CWST value of the filter material can be improved by leading hydrophilic monomer or polymer into the surface of the filter material using a surface improving technique such as graft polymerization, coating, chemical treatment, or plasma treatment.

A priming method according to this embodiment is explained with reference to Figure 7. The priming method is executed using the priming system 20 explained above. When priming is performed, the second port 4 is arranged in a position lower than the first port 3 in the leukocyte remover 12. In particular, in this embodiment, the leukocyte remover 12 is placed substantially vertically such that the second port 4 is located in the lowest position of the leukocyte remover 12 and the first port 3 is located in the highest position of the leukocyte remover 12. When the first port 3 and the second port 4 are arranged in this way and the physiological solution is led in from the second port 4, the physiological solution flows from the lower side to the upper side in the vertical direction on the inside of the leukocyte remover 12. Therefore, the air on the inside of the leukocyte remover 12 is pushed out from the first port 3 located in the highest position of the leukocyte remover. When the leukocyte remover 12 is placed substantially vertically, the air on the inside is successfully exhausted. However, even if the leukocyte remover 12 is inclined 10 degrees from the vertical direction, a substantial problem does not occur in the exhaust.

When the second port side space 8 of the leukocyte remover 12 is filled with the physiological solution, the flowing-in physiological solution should not have pulsation. If the liquid surface of the physiological solution is moved up and down by pulsation, it is likely that the filter 5 present in a position higher than the liquid surface is wet to lose air permeability and the air remains in the second port side space 8.

The opening and closing means 10 opens the channel of the circuit 11, whereby the physiological solution stored in the storing section 9 is led into the leukocyte remover 12 using head pressure without using a pump. Therefore, the storing section 9 for the physiological solution is arranged in a position higher than the leukocyte remover 12. The difference in elevation between the storing section 9 for the physiological solution and the leukocyte remover 12 may be any difference in elevation as long as the difference in elevation can generate head pressure with which average liquid surface rising speed of the physiological solution in the second port side space 8 is equal to or higher than the water absorption speed of the filter 5.

The average liquid surface rising speed refers to liquid surface rising speed of the physiological solution measured as explained below. First, the leukocyte remover 12 is erected substantially vertically such that the second port 4 is located in the lowest position of the leukocyte remover 12 and the first port 3 is located in the highest position of the leukocyte remover 12. The physiological solution is led in from the second port 4. Time T₁ (sec) from time when the liquid surface of the physiological solution reaches the second port 4 until the liquid surface of the physiological solution rises while the physiological solution filling the second port side space 8 and reaches height H (mm), which is 50% of the length from the distal end of the second port 4 to the distal end of the first port 3, is measured. A value (mm/sec) obtained by dividing the height H by the time T₁ is set as the average liquid surface rising speed.

The water absorption speed of the filter 5 is measured as explained below. The filter material included in the filter 5 is cut into a strip shape having width of 10 mm and length of 40 mm. The strip is set substantially vertically. A portion to 10 mm above the bottom of the strip is immersed in the physiological solution used for priming. The front of liquid formed by infiltration of the physiological solution into the strip is observed. Time T₂ (sec) from time when the leading end of the front reaches 20 mm above the bottom of the strip until the leading end of the front reaches 30 mm above the bottom of the strip is measured. A value (mm/sec) obtained by dividing a moving distance 10 mm of the liquid surface by the time T₂ is set as the water absorption speed of the filter 5. When the filter 5 includes plural filter materials, water absorption speeds are measured for the respective filter materials. The water absorption speed of the filter material having the highest water absorption speed is set as the water absorption speed of the filter 5.

The average liquid surface rising speed of the physiological solution has to be equal to or higher than the water absorption speed of the filter 5. If the average liquid surface rising speed of the physiological solution is equal to or lower than the water absorption speed of the filter 5, the entire surface of the filter 5 gets wet before the physiological solution fills the second port side space 8. The air left in the second port side space 8 cannot pass through the wet filter 5 and is trapped in the second port side space 8. If the average liquid surface rising speed of the physiological solution is equal to or higher than the water absorption speed of the filter 5, the air in the second port side space 8 passes through the filter 5 in the dry state and is exhausted before the entire surface of the filter 5 gets wet. The average liquid surface rising speed of the physiological solution only has to be equal to or higher than the water absorption speed of the filter 5. However, the average liquid surface rising speed is desirably equal to or higher than 1.5 times or more of the water absorption speed, more desirably equal to or higher than two times or more of the water absorption speed, and still more desirably three times or more of the water absorption speed.

The air in the second port side space 8 is desirably completely exhausted. However, if a volume occupied by the air is within 10% of the second port side space 8, this is allowable because influence on filtering efficiency of blood is little. If the volume occupied by the air in the second port side space 8 is equal to or larger than 10%, this is undesirable because an effective filtering area of the filter 5 decreases and the filtering efficiency of blood falls.

To set the average liquid surface rising speed of the physiological solution to be equal to or higher than the water absorption speed of the filter 5, the difference in elevation between the storing section 9 for the physiological solution and the leukocyte remover 12 and the channel diameter of the circuit 11 that connects the storing section 9 and the leukocyte remover 12 only have to be adjusted such that, if the channel is fully opened by the opening and closing means 10, the average liquid surface rising speed of the physiological solution is equal to or higher than the water absorption speed of the filter 5. Alternatively, the circuit 11 may be partially opened using the opening and closing means 10 such as a roller clamp, which can be attached to the circuit 11 and adjust the channel diameter of the circuit 11, such that the average liquid surface rising speed of the physiological solution is equal to or higher than the water absorption speed of the filter 5.

Since the air in the first port side space 7 directly communicates with the first port 3, which is the outlet of the air, there is no particular problem in exhausting the air in the first port side space 7.

With the priming method for the leukocyte remover 12 and the priming system 20 according to this embodiment explained above, in priming treatment in the filter 5 in a substantially dry state, it is possible to remove the air remaining in the container such as the flat container 1 or the cylindrical container 2, in which the filter 5 is housed, and prevent deterioration in filter performance.

### Examples

The present invention is explained more in detail using examples explained below. However, the present invention is not limited by the examples.

### [Example 1]

2-hydroxyethyl methacrylate (HEMA) and dimethylaminoethyl methacrylate (DM) were mixed at a ratio of 97:3 in a molar ratio. Total monomer concentration in ethanol was set to 1.0 mol/L. Normal solution radical polymerization was performed at 60°C for eight hours under the presence of a polymerization initiator of 1/200 mol/L of azobisisobutyronitrile, whereby a copolymer (hereinafter abbreviated as HM-3) was composed.

In a solution obtained by dissolving this HM-3 polymer with a 50% ethanol water solution to have polymer concentration of 0.8 weight %, nonwoven fabric made of polyethylene terephthalate having an average fiber diameter of 2.3 µm, weight per unit area 60 g/m², and thickness of 0.30 mm was immersed. After removing excess liquid, the nonwoven fabric was dried at 50°C for twenty minutes to obtain a filter material (A).

The obtained filter material (A) was cut into a 100 mm square. Ten drips each of sodium hydroxide water solutions each having a difference in surface tension of 2 dyn/cm in a range of 90 dyn/cm to 100 dyn/cm were placed on the filter material (A) in order from the sodium hydroxide water solution having the lowest surface tension and left untouched for ten minutes. After being left untouched for ten minutes, the filter material (A) was wet by the sodium hydroxide water solution having surface tension of 94 dyn/cm and not wet by the sodium hydroxide water solution having surface tension of 96 dyn/cm. Therefore, a CWST value of the filter material (A) was 95 dyn/cm.

The obtained filter material (A) was cut into a sample having width of 10 mm and length of 40 mm and the cut sample was suspended in the vertical direction with an upper part of the cut sample fixed by a clip. When the cut sample was immersed in physiological saline up to 10 mm from the bottom and was visually observed, time from time when the physiological saline infiltrated the cut sample up to height of 20 mm from the bottom of the cut sample until the physiological saline infiltrated the cut sample up to height of 30 mm from the bottom of the cut sample was measured. Further, when the height of 10 mm of an infiltration observation range was divided by the infiltration time (sec), water absorption speed of the filter material (A) was 2.0 mm/sec.

Subsequently, in a solution obtained by dissolving the HM-3 polymer with the 50% ethanol water solution to have polymer concentration of 0.8 weight %, nonwoven fabric made of polyethylene terephthalate having an average fiber diameter of 12 µm, weight per unit area 100 g/m², and thickness of 0.47 mm was immersed. After removing excess liquid, the nonwoven fabric was dried at 50°C for twenty minutes to obtain a filter material (B).

The obtained filter material (B) was cut into a 100 mm square. Ten drips each of sodium hydroxide water solutions each having a difference in surface tension of 2 dyn/cm in a range of 90 dyn/cm to 100 dyn/cm were placed on the filter material (B) in order from the sodium hydroxide water solution having the lowest surface tension and left untouched for ten minutes. After being left untouched for ten minutes, the filter material (B) was wet by the sodium hydroxide water solution having surface tension of 94 dyn/cm and not wet by the sodium hydroxide water solution having surface tension of 96 dyn/cm. Therefore, a CWST value of the filter material (B) was 95 dyn/cm.

The obtained filter material (B) was cut into a sample having width of 10 mm and length of 40 mm and the cut sample was suspended in the vertical direction with an upper part of the cut sample fixed by a clip. When the cut sample was immersed in physiological saline up to 10 mm from the bottom and was visually observed, time from time when the physiological saline infiltrated the cut sample up to height of 20 mm from the bottom of the cut sample until the physiological saline infiltrated the cut sample up to height of 30 mm from the bottom of the cut sample was measured. Further, when the height of 10 mm of an infiltration observation range was divided by the infiltration time (sec), water absorption speed of the filter material (B) was 2.5 mm/sec.

Eighteen filter materials (A) were laminated and ten filter materials (B) were laid over the filter materials (A) to create a sheet-like filter. The water absorption speed of the sheet-like filter was set to the water absorption speed 2.5 mm/sec of the filter material (B).

The sheet-like filter was cut into a 97 mm square and loaded in a square flat container with a capacity of 125 mL, which had a first port and a second port for liquid respectively at opposed corner portions, such that the filter materials (A) were located on the second port side. Ultrasonic welding was performed to manufacture a flat leukocyte remover.

The flat leukocyte remover was arranged to be erected substantially vertically such that the second port was located in the lowest position and the first port was located in the highest position. The second port and a physiological saline bag (storing section) were connected by a circuit with roller clamp. The physiological saline bag was fixed in a position higher than the flat leukocyte remover. The roller clamp was adjusted to set average liquid surface rising speed in a second port side space to 4.3 mm/sec. The inside of the flat leukocyte remover was primed by 500 mL of the physiological saline. When a volume occupied by the air in the second port side space after the end of the priming was measured and a ratio of the volume occupied by the air in the second port side space was calculated, the ratio was 2.6%.

### [Comparative Example 1]

A flat leukocyte remover manufactured by the same method as the example 1 was used. The flat leukocyte remover was primed by the same method as the example 1 except that the roller clamp was adjusted to set the average liquid surface rising speed to 1.8 mm/sec. After the priming ends, when a ratio of a volume occupied by the air in the second port side space was calculated, the ratio was 16.9%.

### [Example 2]

The HM-3 polymer was composed by the same method as the example 1. The HM-3 polymer was dissolved by a 50% ethanol water solution to have polymer concentration of 0.2 weight %. In this solution, nonwoven fabric made of polyethylene terephthalate having an average fiber diameter of 1.1 µm, weight per unit area 40 g/m², and thickness of 0.23 mm was immersed. After removing excess liquid, the nonwoven fabric was dried at 110°C for one minute to obtain a filter material (C).

The obtained filter material (C) was cut into a 100 mm square. Ten drips each of sodium hydroxide water solutions each having a difference in surface tension of 2 dyn/cm in a range of 80 dyn/cm to 90 dyn/cm were placed on the filter material (C) in order from the sodium hydroxide water solution having the lowest surface tension and left untouched for ten minutes. After being left untouched for ten minutes, the filter material (C) was wet by the sodium hydroxide water solution having surface tension of 86 dyn/cm and not wet by the sodium hydroxide water solution having surface tension of 88 dyn/cm. Therefore, a CWST value of the filter material (C) was 87 dyn/cm.

The obtained filter material (C) was cut into a sample having width of 10 mm and length of 40 mm and the cut sample was suspended in the vertical direction with an upper part of the cut sample fixed by a clip. When the cut sample was immersed in physiological saline up to 10 mm from the bottom and was visually observed, time from time when the physiological saline infiltrated the cut sample up to height of 20 mm from the bottom of the cut sample until the physiological saline infiltrated the cut sample up to height of 30 mm from the bottom of the cut sample was measured. Further, when the height of 10 mm of an infiltration observation range was divided by the infiltration time (sec), water absorption speed of the filter material (C) was 0.8 mm/sec.

Subsequently, twenty-eight filter materials (C) were laminated to manufacture a sheet-like filter. Water absorption speed of the sheet-like filter was set to the water absorption speed 0.8 mm/sec of the filter material (C).

The sheet-like filter was cut into a 62 mm square and loaded in a square flat container with a capacity of 16 mL, which had a first port and a second port for liquid respectively at opposed corner portions. Ultrasonic welding was performed to manufacture a flat leukocyte remover.

The flat leukocyte remover was arranged in the vertical direction such that the second port was located in the lowest position and the first port was located in the highest position. The second port and a physiological saline bag were connected by a circuit with roller clamp. The physiological saline bag was fixed in a position higher than the flat leukocyte remover. The roller clamp was adjusted to set average liquid surface rising speed in a second port side space to 2.5 mm/sec. The inside of the flat leukocyte remover was primed by 500 mL of the physiological saline. When a volume occupied by the air in the second port side space after the end of the priming was measured and a ratio of the volume occupied by the air in the second port side space was calculated, the ratio was 1.1 %.

### [Comparative Example 2]

A flat leukocyte remover manufactured by the same method as the example 2 was used. The flat leukocyte remover was primed by the same method as the example 1 except that the roller clamp was adjusted to set the average liquid surface rising speed to 0.1 mm/sec. After the priming ends, when a ratio of a volume occupied by the air in the second port side space was calculated, the ratio was 43.0%.

### [Example 3]

An ethylene-vinylalcohol copolymer (hereinafter abbreviated as EVOH. Ethylene content is 29%: manufactured by The Nippon Synthetic Chemical Industry Co., Ltd.) is dissolved in a 58% propanol water solution to have polymer concentration of 1.0 weight %. In this solution, nonwoven fabric made of polyethylene terephthalate having an average fiber diameter of 1.8 µm, weight per unit area 66 g/m², and thickness of 0.42 mm was immersed. After removing excess liquid, the nonwoven fabric was dried at 50°C for twenty minutes to obtain a filter material (D).

The obtained filter material (D) was cut into a 100 mm square. Ten drips each of sodium hydroxide water solutions each having a difference in surface tension of 2 dyn/cm in a range of 80 dyn/cm to 90 dyn/cm were placed on the filter material (D) in order from the sodium hydroxide water solution having the lowest surface tension and left untouched for ten minutes. After being left untouched for ten minutes, the filter material (D) was wet by the sodium hydroxide water solution having surface tension of 86 dyn/cm and not wet by the sodium hydroxide water solution having surface tension of 88 dyn/cm. Therefore, a CWST value of the filter material (D) was 87 dyn/cm.

The obtained filter material (D) was cut into a sample having width of 10 mm and length of 40 mm and the cut sample was suspended in the vertical direction with an upper part of the cut sample fixed by a clip. When the cut sample was immersed in physiological saline up to 10 mm from the bottom and was visually observed, time from time when the physiological saline infiltrated the cut sample up to height of 20 mm from the bottom of the cut sample until the physiological saline infiltrated the cut sample up to height of 30 mm from the bottom of the cut sample was measured. Further, when the height of 10 mm of an infiltration observation range was divided by the infiltration time (sec), water absorption speed of the filter material (D) was 1.1 mm/sec.

Subsequently, in a solution obtained by dissolving the EVOH with the 58% propanol water solution to have polymer concentration of 0.3 weight %, nonwoven fabric made of polyethylene terephthalate having an average fiber diameter of 12 µm, weight per unit area 100 g/m², and thickness of 0.47 mm was immersed. After removing excess liquid, the nonwoven fabric was dried at 50°C for twenty minutes to obtain a filter material (E).

The obtained filter material (E) was cut into a 100 mm square. Ten drips each of sodium hydroxide water solutions each having a difference in surface tension of 2 dyn/cm in a range of 80 dyn/cm to 90 dyn/cm were placed on the filter material (E) in order from the sodium hydroxide water solution having the lowest surface tension and left untouched for ten minutes. After being left untouched for ten minutes, the filter material (E) was wet by the sodium hydroxide water solution having surface tension of 86 dyn/cm and not wet by the sodium hydroxide water solution having surface tension of 88 dyn/cm. Therefore, a CWST value of the filter material (E) was 87 dyn/cm.

The obtained filter material (E) was cut into a sample having width of 10 mm and length of 40 mm and the cut sample was suspended in the vertical direction with an upper part of the cut sample fixed by a clip. When the cut sample was immersed in physiological saline up to 10 mm from the bottom and was visually observed, time from time when the physiological saline infiltrated the cut sample up to height of 20 mm from the bottom of the cut sample until the physiological saline infiltrated the cut sample up to height of 30 mm from the bottom of the cut sample was measured. Further, when the height of 10 mm of an infiltration observation range was divided by the infiltration time (sec), water absorption speed of the filter material (E) was 0.8 mm/sec.

Subsequently, four filter materials (D) were laminated and ten filter materials (E) were laid over the filter materials (D) to create a sheet-like filter. The water absorption speed of the sheet-like filter was set to the water absorption speed 1.1 mm/sec of the filter material (D).

The sheet-like filter was cut into a circle having a diameter of 110 mm and loaded in a circular flat container with a capacity of 43 mL, which had a first port and a second port for liquid respectively at opposed circumferential edge portions, such that the filter materials (D) were located on the second port side. The circumferential edge portion of the container was closed by a screw lamp to manufacture a flat leukocyte remover.

The flat leukocyte remover was arranged in the vertical direction such that the second port was located in the lowest position and the first port was located in the highest position. The second port and a physiological saline bag were connected by a circuit with roller clamp. The physiological saline bag was fixed in a position higher than the flat leukocyte remover. The roller clamp was adjusted to set average liquid surface rising speed in a second port side space to 45.2 mm/sec. The inside of the flat leukocyte remover was primed by 500 mL of the physiological saline. When a volume occupied by the air in the second port side space after the end of the priming was measured and a ratio of the volume occupied by the air in the second port side space was calculated, the ratio was 0%.

### [Comparative Example 3]

A flat leukocyte remover manufactured by the same method as the example 3 was used. The flat leukocyte remover was primed by the same method as the example 1 except that the roller clamp was adjusted to set the average liquid surface rising speed to 1.0 mm/sec. After the priming ends, when a ratio of a volume occupied by the air in the second port side space was calculated, the ratio was 42.7%.

### [Example 4]

The filter material (A) obtained in the same manner as the example 1 was cut into a filter material having width of 150 mm and length of 1000 mm and the cut filter material was wound around a cylindrical mesh made of polyethylene having a diameter of 20 mm. The filter material (B) cut into a filter material having width of 150 mm and length of 500 mm was wound and laminated on the outer side of the filter material wound around the mesh. A mesh made of polyethylene having width of 150 mm and length of 130 mm was wound further on the outer side to create a cylindrical filter. The water absorption speed of the cylindrical filter was set to the water absorption speed 2.5 mm/sec of the filter material (B).

Both ends of the cylindrical filter were closed by urethane. The cylindrical filter was loaded in a cylindrical container with a capacity of 198 mL, which had a first port and a second port for liquid respectively in opposed surface portions, such that the inner circumferential surface of the cylindrical filter was located on the first port side and the outer circumferential side of the cylindrical filter was located on the second port side. In this way, a cylindrical leukocyte remover was manufactured.

The cylindrical leukocyte remover was arranged in the vertical direction such that the second port was located in the lowest position and the first port was located in the highest position. The second port and a physiological saline bag were connected by a circuit with roller clamp. The physiological saline bag was fixed in a position higher than the flat leukocyte remover. The roller clamp was adjusted to set average liquid surface rising speed in a second port side space to 5.7 mm/sec. The inside of the cylindrical leukocyte remover was primed by 500 mL of the physiological saline. When a volume occupied by the air in the second port side space after the end of the priming was measured and a ratio of the volume occupied by the air in the second port side space was calculated, the ratio was 4.5%.

### [Comparative Example 4]

A cylindrical leukocyte remover manufactured by the same method as the example 4 was used. The cylindrical leukocyte remover was primed by the same method as the example 1 except that the roller clamp was adjusted to set the average liquid surface rising speed to 1.2 mm/sec. After the priming ends, when a ratio of a volume occupied by the air in the second port side space was calculated, the ratio was 38.2%.

The results explained above are tabulated in Table 1.

**[Table 1]**

| Item | Example 1 | Comparative example 1 | Example 2 | Comparative example 2 | Example 3 | Comparative example 3 | Example 4 | Comparative example 4 |
|---|---|---|---|---|---|---|---|---|
| Base material | PET | PET | PET | PET | PET | PET | PET | PET |
| Shape | Nonwoven fabric | Nonwoven fabric | Nonwoven fabric | Nonwoven fabric | Nonwoven fabric | Nonwoven fabric | Nonwoven fabric | Nonwoven fabric |
| Coat polymer | HM-3 | HM-3 | HM-3 | HM-3 | EVOH | EVOH | HM-3 | HM-3 |
| Filter water absorption speed (mm/sec) | 2.5 | 2.5 | 0.8 | 0.8 | 1.1 | 1.1 | 2.5 | 2.5 |
| Filter shape | Sheet-like | Sheet-like | Sheet-like | Sheet-like | Sheet-like | Sheet-like | Cylindrical | Cylindrical |
| Container shape | Square flat | Square flat | Square flat | Square flat | Circular flat | Circular flat | Circular cylindrical | Circular cylindrical |
| Container capacity (mL) | 125 | 125 | 16 | 16 | 43 | 43 | 198 | 198 |
| Average liquid surface rising speed (mm/sec) | 4.3 | 1.8 | 2.5 | 0.1 | 45.2 | 1.0 | 5.7 | 1.2 |
| Ratio of remaining air volume (%) | 2.6 | 16.9 | 1.1 | 43.0 | 0 | 42.7 | 4.5 | 38.2 |

### Industrial Applicability

According to the present invention, it is possible to provide a system that can simply prime, without leaving an amount of the air problematic in treatment, a leukocyte remover in which a filter in a substantially dry state is loaded.

### Reference Signs List

1, 1A, 1B, 1C ... flat containers
2, 2A, 2B, 2C ... cylindrical containers
3 ... first port
4 ... second port
5, 5A, 5B, 5C ... filters
6 ... adhesive
7 ... first port side space
8 ... second port side space
9 ... storing section for physiological solution
10 ... opening and closing means
11 ... circuit
12 ... leukocyte remover
20 ... priming system

## Claims

1. A method of priming a leukocyte remover (12) in which a filter (5; 5A; 5B; 5C) in a substantially dry state is housed in a container (1; 2; 1 A; 1B; 1C; 2A; 2B; 2C) having a first port (3) and a second port (4) for liquid provided near a peripheral edge thereof and a space in the container is partitioned into a first port side space (7) and a second port side space (8) by the filter (5; 5A; 5B; 5C), the priming method comprising:
arranging the leukocyte remover (12) such that the second port (4) is located in a lowest position of the leukocyte remover and the first port (3) is located in a highest position of the leukocyte remover by placing the leukocyte remover substantially vertically but whereby the leukocyte remover can be inclined by up to 10 degrees from the vertical direction;
leading in a physiological solution from the second port (4); and setting the average liquid surface rising speed of the physiological solution in the second port side space (8) to be equal to or higher than the water absorption speed of the filter (5; 5A; 5B; 5C).

2. The priming method according to claim 1, wherein the physiological solution is stored in a position higher than the leukocyte remover (12) and led in from the second port (8) with head pressure.

3. The priming method according to claim 1 or 2, wherein critical wet surface tension of the filter (5; 5A; 5B; 5C) is equal to or larger than surface tension of the physiological solution.

4. The priming method according to any one of claims 1 to 3, wherein the container is a flat container (1; 1A; 1B; 1C) or a cylindrical container (2; 2A; 2B; 2C).

5. The priming method according to any one of claims 1 to 4, wherein the filter (5; 5A; 5B; 5C) is formed of any one of nonwoven fabric, woven fabric, and a porous body.

6. The priming method according to claim 4, wherein a shape of the flat container or the cylindrical container is a polygonal shape (1B; 1C; 2B; 2C) or a circular shape (1A; 2A).

## Patentansprüche

1. Verfahren zum Vorbereiten eines Leukocytenentferners (12), bei dem ein Filter (5; 5A; 5B; 5C) in einem im Wesentlichen trockenen Zustand in einem Behälter (1; 2; 1A; 1B; 1C; 2A; 2B; 2C), der einen ersten Anschluss (3) und einen zweiten Anschluss (4) aufweist, untergebracht ist, für Flüssigkeit, die sich in der Nähe von seinem Umfangsrand befindet, und ein Raum im Behälter durch den Filter (5; 5A; 5B; 5C) in einen Raum auf der Seite des ersten Anschlusses (7) und einen Raum auf der Seite des zweiten Anschlusses (8) unterteilt ist, wobei das Vorbereitungsverfahren umfasst:
Anordnen des Leukocytenentferners (12) in einer solchen Weise, dass sich der zweite Anschluss (4) in einer tiefsten Position des Leukocytenentferners befindet und sich der erste Anschluss (3) in einer höchsten Position des Leukocytenentferners befindet, indem man den Leukocytenentferner im Wesentlichen senkrecht platziert, wobei der Leukocytenentferner jedoch gegenüber der vertikalen Richtung um bis zu 10 Grad geneigt sein kann;
Einleiten einer physiologischen Lösung aus dem zweiten Anschluss (4); und
Einstellen der durchschnittlichen Flüssigkeitsspiegel-Anstiegsgeschwindigkeit der physiologischen Lösung im Raum auf der Seite des zweiten Anschlusses (8) auf einen solchen Wert, dass sie größer oder gleich der Wasseraufsauggeschwindigkeit des Filters (5; 5A; 5B; 5C) ist.

2. Vorbereitungsverfahren gemäß Anspruch 1, wobei die physiologische Lösung in einer höheren Position als der Leukocytenentferner (12) aufbewahrt und unter Ausstoßdruck aus dem zweiten Anschluss (8) eingeleitet wird.

3. Vorbereitungsverfahren gemäß Anspruch 1 oder 2, wobei die kritische Nassoberflächenspannung des Filters (5; 5A; 5B; 5C) größer oder gleich der Oberflächenspannung der physiologischen Lösung ist.

4. Vorbereitungsverfahren gemäß einem der Ansprüche 1 bis 3, wobei der Behälter ein flacher Behälter (1; 1A; 1B; 1C) oder ein zylindrischer Behälter (2; 2A; 2B; 2C) ist.

5. Vorbereitungsverfahren gemäß einem der Ansprüche 1 bis 4, wobei der Filter (5; 5A; 5B; 5C) aus einem Vliesstoff, einem Gewebe oder einem porösen Körper gebildet ist.

6. Vorbereitungsverfahren gemäß Anspruch 4, wobei die Form des flachen Behälters oder des zylindrischen Behälters eine Vieleckform (1B; 1C; 2B; 2C) oder eine Kreisform (1A; 2A) ist.

## Revendications

1. Procédé d'amorçage d'un système d'élimination de leucocytes (12) dans lequel un filtre (5 ; 5A ; 5B ; 5C) dans un état sensiblement sec est logé dans un récipient (1 ; 2 ; 1A ; 1B ; 1C ; 2A ; 2B ; 2C) ayant un premier orifice (3) et un second orifice (4) pour liquide prévu à proximité d'un bord périphérique de celui-ci et un espace dans le récipient est séparé en un espace côté premier orifice (7) et un espace côté second orifice (8) par le filtre (5 ; 5A ; 5B ; 5C), le procédé d'amorçage comprenant :
l'agencement du système d'élimination de leucocytes (12) de telle sorte que le second orifice (4) se trouve dans une position la plus basse du système d'élimination de leucocytes et que le premier orifice (3) se trouve dans une position la plus haute du système d'élimination de leucocytes en mettant en place le système d'élimination de leucocytes sensiblement verticalement tout en permettant au système d'élimination de leucocytes d'être incliné jusqu'à 10 degrés par rapport à la direction verticale ;
l'introduction d'une solution physiologique à partir du second orifice (4) ; et
le réglage de la vitesse de montée de surface de liquide moyenne de la solution physiologique dans l'espace côté second orifice (8) pour qu'elle soit supérieure ou égale à la vitesse d'absorption d'eau du filtre (5 ; 5A ; 5B ; 5C).

2. Procédé d'amorçage selon la revendication 1, dans lequel la solution physiologique est stockée dans une position plus haute que le système d'élimination de leucocytes (12) et introduite à partir du second orifice (8) avec pression de refoulement.

3. Procédé d'amorçage selon la revendication 1 ou 2, dans lequel une tension superficielle à l'état humide critique du filtre (5 ; 5A ; 5B ; 5C) est supérieure ou égale à une tension superficielle de la solution physiologique.

4. Procédé d'amorçage selon l'une quelconque des revendications 1 à 3, dans lequel le récipient est un récipient plat (1 ; 1A ; 1B ; 1C) ou un récipient cylindrique (2 ; 2A ; 2B ; 2C) ;

5. Procédé d'amorçage selon l'une quelconque des revendications 1 à 4, dans lequel le filtre (5 ; 5A ; 5B ; 5C) est formé de l'un quelconque d'un tissu non tissé, d'un tissu tissé et d'un corps poreux.

6. Procédé d'amorçage selon la revendication 4, dans lequel une forme du récipient plat ou du récipient cylindrique est une forme polygonale (1B ; 1C ; 2B ; 2C) ou une forme circulaire (1A ; 2A).
